# EUROPÄISCHE PATENTSCHRIFT

(11) **EP 1 157 031 B1**
(45) Veröffentlichungstag und Bekanntmachung des Hinweises auf die Patenterteilung: **17.12.2003**
(21) Anmeldenummer: 00912543.6
(22) Anmeldetag: 03.03.2000
(51) Int. Cl.: C07K 14/00, C07K 5/06

(54) **MIT PHOSPHONSÄUREESTER-, PHOSPHONSÄURE- ODER CARBABORAN-FUNKTIONEN SUBSTITUIERTE OLIGOMERE UND DIE ENTSPRECHENDEN PNA-MONOMERE**
OLIGOMERS SUBSTITUTED BY PHOSPHONIC ACID ESTER, PHOSPHONIC ACID OR CARBABORANE FUNCTIONS AND THE CORRESPONDING PNA MONOMERS
OLIGOMERES SUBSTITUES PAR DES FONCTIONS ESTER D'ACIDE PHOSPHONIQUE, ACIDE PHOSPHONIQUE OU CARBABORANE ET MONOMERES PNA CORRESPONDANTS

(30) Priorität: 03.03.1999 DE 19909373
(43) Veröffentlichungstag der Anmeldung: 28.11.2001
(73) Patentinhaber: Ugichem GmbH, 6020 Innsbruck (AT)
(72) Erfinder: BOCK, Holger, D-80807 München (DE); LINDHORST, Thomas, D-81377 München (DE)
(74) Vertreter: Forstmeyer, Dietmar, Dr. rer. nat., Dipl.-Chem.
(86) Internationale Anmeldenummer: EP0001852
(87) Internationale Veröffentlichungsnummer: WO00052038

(56) Entgegenhaltungen:
- DE-A- 19 508 923
- DE-A- 19 640 974

## Beschreibung

Die Erfindung betrifft neue Oligomere, die mit Phosphonsäureester-, Phosphonsäure- oder Carbaboran-Funktionen substituierte PNA-Einheiten enthalten sowie mit Phosphonsäureester-, Phosphonsäure- oder Carbaboran-Funktionen substituierte PNA-Monomere, aus denen die neuen Oligomere hergestellt werden.

Es ist bekannt, daß Peptidnukleinsäuren (PNAs) mit höherer Affinität an komplementäre Nukleinsäuren (DNA oder RNA) als ihre natürlichen Vorbilder binden können (M. Egholm, O. Buchardt, L. Christensen, C. Behrens, S.M. Freier, D.A. Driver, R.H. Berg, S.K. Kim, B. Norden, P.E. Nielsen, Nature, **1993,** 365, 566-568. B. Hyrup, P.E. Nielsen, *Bioorg. Med. Chem*., **1996,** 4, 5-23).

Die Zellgängigkeit der bisher bekannten PNA-Oligomere ist aber im Gegensatz zu DNA bzw. RNA sehr gering. Der praktische Nutzen von PNAs als Antisense-Wirkstoffe hängt aber maßgeblich von deren intrazellulären Verfügbarkeit ab.

Es ist daher Aufgabe der vorliegenden Erfindung, Oligomere bereitzustellen, die wie PNAs an DNAs oder RNAs binden können, dabei aber eine verbesserte Zellgängigkeit aufweisen.

Diese Aufgabe wird erfindungsgemäß gelöst von Verbindungen der Formel

W-U-Z

wobei W ein H-Atom, eine Aminosäure- oder PNA-Einheit sein kann.

U enthält mindestens eine Einheit der Formel Y und gegebenenfalls eine oder mehrere Aminosäure- und/oder PNA-Einheiten.

Z kann eine OH-Funktion, eine Aminosäure-, oder PNA-Einheit sein.

Die Erfinder haben nämlich gefunden, daß vor allem die Einführung einer oder mehrerer Phosphonsäure- bzw. Phosphonsäureester-Funktionen, aber auch die Einführung einer oder mehrerer Carbaboran-Funktionen in die Seitenkette zu einer Erhöhung der Zellgängigkeit von PNA- oder PNA-analogen Oligomeren führt.

Y ist eine Einheit der Formel 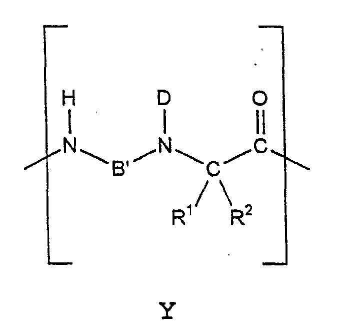 ,worin
B' eine Gruppe der Formel, 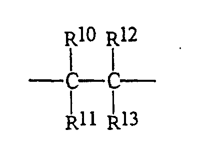 D eine Gruppe der Formel, 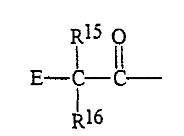 ist.

Die Reste R¹⁰ bis R¹³ können jeweils unabhängig voneinander bis zu 20 C-Atome, bevorzugt 1, 2, 3, 4, 5, 6, 7, 8, 9 oder 10 C-Atome umfassen. Sie können unabhängig voneinander H-Atome, unsubstituierte Alkyl-, Alkenyl-, Alkaryl-, Aryl-, oder alicyclische Reste sein, wobei die Reste verzweigt oder unverzweigt sind, bevorzugt sind diese Reste H-Atome.

Optional können jeweils zwei der Reste R¹⁰ bis R¹³, die durch bis zu zwei Kohlenstoffatome voneinander getrennt sind, Bestandteile eines gemeinsamen Ringsystems sein, wobei dieses Ringsystem ein unsubstituierter oder mit einem verzweigten oder unverzweigten C₁-C₅ Alkylrest substituierter alicyclischer Monocyclus (3-8 Ringatome) oder ein Phenyl-Ring ist, bevorzugt ist dieses Ringsystem ein unsubstituierter Cyclopentyl-, Cyclohexyl- oder Phenyl-Ring.

Die Reste R¹⁵ und R¹⁶ können jeweils unabhängig voneinander bis zu 20 C-Atome, bevorzugt 1, 2, 3, 4, 5, 6, 7, 8, 9 oder 10 C-Atome umfassen. Sie werden unabhängig voneinander ausgewählt aus H-Atomen, unsubstituierten Alkyl-, Alkenyl-, Alkaryl-, Aryl-, oder alicyclischen Resten, wobei die Reste verzweigt oder unverzweigt sind, noch stärker bevorzugt sind diese Reste H-Atome.

Optional können die Reste R¹⁵ und R¹⁶ Bestandteile eines gemeinsamen Ringsystems sein, wobei dieses Ringsystem ein unsubstituierter oder mit einem verzweigten oder unverzweigten C₁-C₅ Alkylrest substituierter alicyclischer Monocyclus (3-6 Ringatome) ist. Bevorzugt ist dieses Ringsystem ein unsubstituierter Cyclohexyloder ein Cyclopentyl-Ring.

In der gesamten Anmeldung können Alkylreste z. B. Methyl-, Ethyl-, Propyl- oder Butyl-Gruppen sein.

E kann eine natürliche oder nichtnatürliche gegebenenfalls mit Schutzgruppen wie X¹ bis X⁴ substituierte Nukleobase sein.

Derartige Nukleobasen sind zur Watson-Crick- oder Hoogsteen-Basenpaarung fähig.
Bevorzugt kann E eine Gruppe der folgenden Formeln 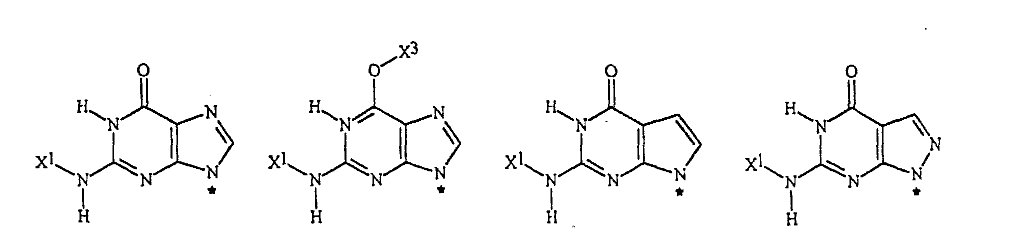 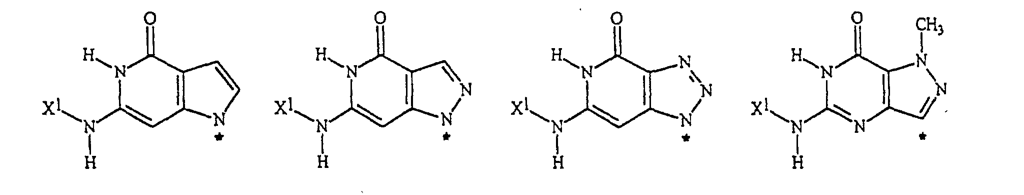 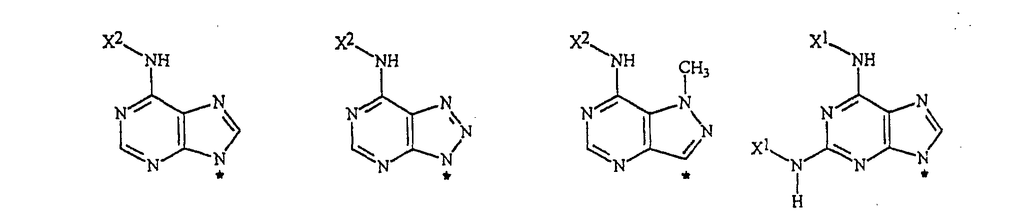 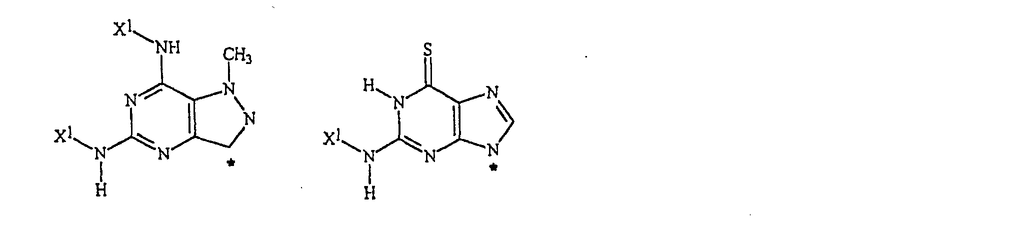 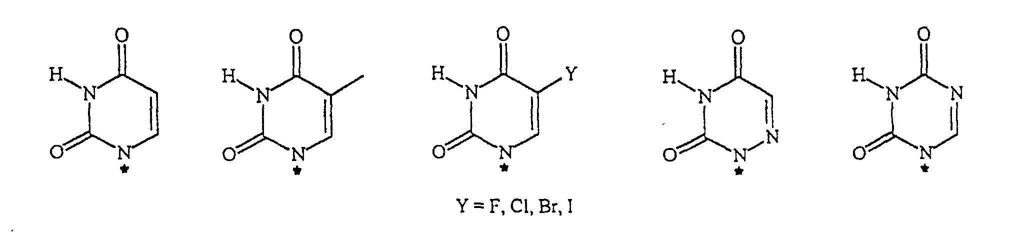 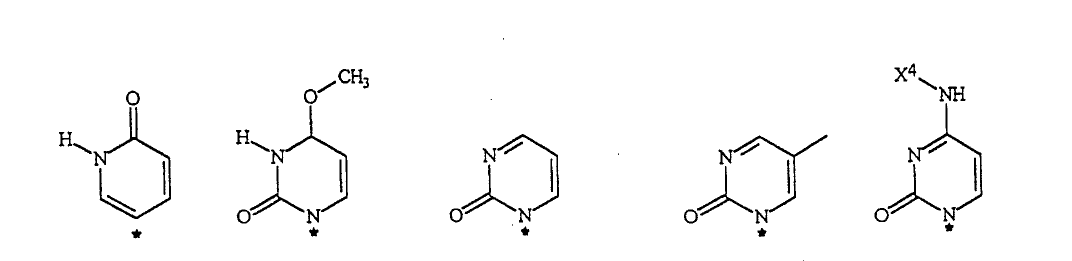 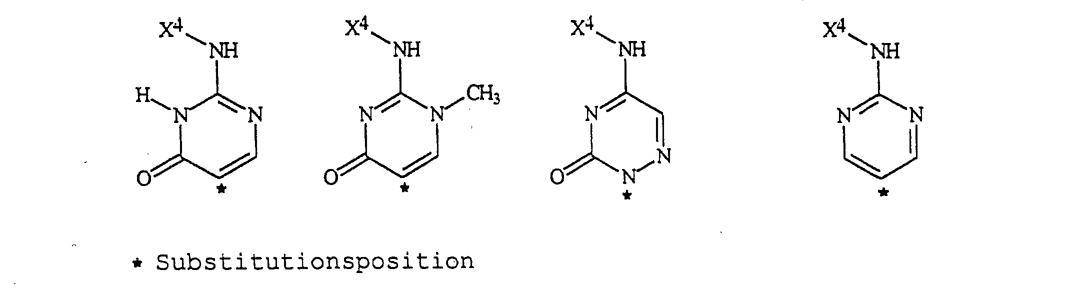 sein, worin X¹ bis X⁴ unabhängig voneinander H-Atome oder die aus der Schutzgruppentechnik für Nukleinbasen bekannten, folgenden Substituenten sein können:
X¹, X², X⁴: Acetyl (Ac) , Isobutyryl (iBu-CO), Benzyloxycarbonyl (Cbz), (4-Methoxyphenyl)-diphenylmethyl (Mmt), Benzhydryloxycarbonyl (Bhoc), Anisoyl (An), 4-tert.-Butylbenzoyl (tBuBz).
X³: Benzyl (Bn), Diphenylcarbamoyl (Dpc).

Noch stärker bevorzugt wird E ausgewählt aus: N²-Acetyl-Guaninyl-, N²-Isobutyryl-Guaninyl-, N²-Benzyloxycarbonyl-Guaninyl-, N²-(4-Methoxyphenyl)-diphenylmethyl-Guaninyl-, N²-Benzhydryloxycarbonyl-Guaninyl-, N⁶-Benzyloxycarbonyl-Adeninyl-, N⁶-(4-Methoxyphenyl)-diphenylmethyl-Adeninyl-, N⁶-Anisoyl-Adeninyl-, N⁶-Benzhydryloxycarbonyl-Adeninyl-, O⁶-Benzylguaninyl- (X¹ ist ein H-Atom) , N²-Acetyl-O⁶-diphenylcarbamoyl-Guaninyl-, N²-Isobutyryl-O⁶-diphenylcarbamoyl-Guaninyl-, N²-Benzyloxycarbonyl-O⁶-diphenylcarbamoyl-Guaninyl-, N²-(4-Methoxyphenyl)-diphenylmethyl-O⁶-diphenylcarbamoyl-Guaninyl-, N²-Benzhydryloxycarbonyl-O⁶-diphenylcarbamoyl-Guaninyl-, N⁴-Benzyloxycarbonyl-Cytosinyl-, N⁴-(4-Methoxyphenyl)-diphenylmethyl-Cytosyl-, N⁴-4-tert.Butylbenzoyl-Cytosinyl-, N⁴-Benzhydryloxycarbonyl-Cytosinyl-, N²-Benzyloxycarbonyl-Pseudoisocytosinyl-, N²-(4-Methoxyphenyl)-diphenylmethyl-Pseudoisocytosinyl-, N²-4-tert.-Butylbenzoyl-Pseudoisocytosinyl-, N²-Benzhydryloxycarbonyl-Pseudoisocytosinyl-, Adeninyl-, Cytosinyl-, Pseudoisocytosinyl-, Guaninyl-, Thyminyl-, oder ein Uracilyl-Rest.
Am stärksten bevorzugt ist E ein Adeninyl-, Cytosinyl-, Pseudoisocytosinyl-, Guaninyl-, Thyminyl- oder ein Uracilyl-Rest.

Die Reste R¹ und R² können jeweils unabhängig voneinander H-Atome substituierte Alkyl-, Alkenyl-, Alkaryl-, Aryl-, oder alicyclische Reste mit bis zu 20 C-Atomen sein, wobei mindestens einer der Reste R¹ oder R² eine oder mehrere Phosphonsäureester-, Phosphonsäure- oder Carbaboran-Funktionen aufweist.

Phosphonsäure-Funktionen können zum Beispiel die Formel -P(=O)(OH)₂ aufweisen.
Phosphonsäureester-Funktionen können zum Beispiel die Formel -P(=O)(OV)₂ oder -P(=O)(OV)(OH) aufweisen. Dabei kann V ein unsubstituierter Alkyl-, Alkenyl-, Alkaryl-, Aryl-, oder alicyclischer Rest mit bis zu 20 C-Atomen, stärker bevorzugt mit bis zu 7 C-Atomen und am stärksten bevorzugt ein Methyl-, Ethyloder Benzyl-Rest sein.

Es werden Carbaboran-Funktionen mit bis zu 20 Boratomen - insbesondere bis zu 12, 10 oder 8 Boratomen - und 1 bis 4 C-Atomen bevorzugt, wobei bekannte Carbaboran-Funktionen besonders bevorzugt werden.

Bevorzugt umfassen die Reste R¹ oder R² 1, 2, 3, 4, 5, 6, 7, 8, 9 oder 10 C-Atome.
Die Reste R¹ oder R² können verzweigt oder unverzweigt sein. Am stärksten bevorzugt sind die Reste R¹ und R² wie oben definiert, wobei zumindest einer der Reste R¹ oder R² einen Substituenten einer nicht natürlichen Aminosäure umfasst oder darstellt.

Am stärksten werden die Reste R¹ und R² unabhängig voneinander aus H-Atomen, oder einer Gruppe der Formeln -CH₂-[P(=O)(O-K)₂] oder -CH₂-C(CH₃)₂-[P(=O)(O-K)₂] ausgewählt, wobei Kein H-Atom, ein Methyl-, Ethyl-, oder ein Benzyl-Rest ist.

PNAs sind gegebenenfalls substituierte Oligomere mit einem N-(2-Aminoethyl)glycin-Backbone. Der Substituent NB stellt eine Nukleobase dar.

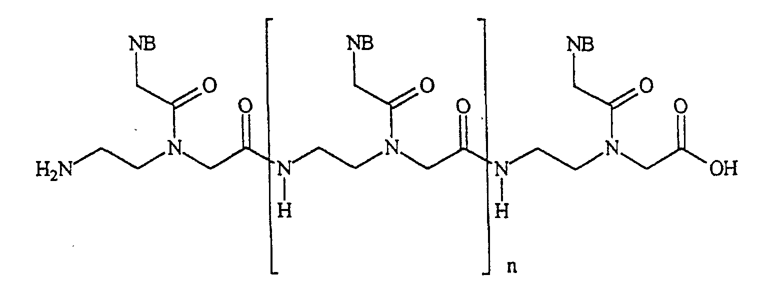

PNA-Oligomere werden durch Knüpfen von Peptidbindungen zwischen substituierten N-Acetyl-N-(2-aminoethyl)glycin-Bausteinen (PNA-Monomere) hergestellt. Im Oligomer stellt jeder einzelne dieser substituierten N-Acetyl-N-(2-aminoethyl)glycin-Bausteine eine PNA-Einheit dar. Erfindungsgemäß können an sich bekannte PNA-Einheiten eingesetzt werden, wobei Einheiten der vorstehend dargestellten Formel bevorzugt werden.

Vorzugsweise ist die Verbindung W-U-Z aus bis zu 50, stärker bevorzugt aus bis zu 40 und am stärksten bevorzugt aus bis zu 30 dieser Einheiten W, U und Z aufgebaut. Zum Beispiel können derartige Verbindungen W-U-Z bis zu 5 Einheiten der Formel W, bis zu 30 Einheiten der Formel U und bis zu 10 Einheiten der Formel Z enthalten.

Stärker bevorzugt ist W ein H-Atom, umfasst U eine oder mehrere Einheiten der Formel Y und eine oder mehrere PNA-Einheiten und ist Z eine OH-Gruppe.

Am stärksten bevorzugt ist W ein H-Atom, U eine oder mehrere Einheiten der Formel Y, und Z eine OH-Gruppe.

Wenn die Oligomere Carbaboran-Funktionen enthalten, können sie im Rahmen der Bor-Neutronen-Einfang-Therapie (BNCT) zur Bekämpfung von Krebstumoren eingesetzt werden. Bei der BNCT werden borhaltige Moleküle in Krebszellen eingeschleust. Die Zellen werden anschließend mit langsamen Neutronen beschossen, wodurch die Bor-Atome in energiereiche Teilchen zerfallen und umliegendes Gewebe irreversibel zerstören (*Chemie in unserer Zeit* **1997**, *31. Jahrg. Nr. 5,* 235). Im Rahmen der BNCT wurden borhaltige Aminosäuren, Zucker, Porphyrine, Phospholipide, Thiouracil-Derivate, Nukleotidanaloga und Nukleoside synthetisiert und untersucht (M. F. Hawthorne, *Angew. Chem*. **1993,** *105*, 997).

Erfindungsgemäß kann U ein durch Aneinanderfügen von Aminosäureund/oder PNA-Einheiten und mindestens einer Einheit der Formel Y in beliebiger Reihenfolge aufgebautes Oligomer sein.

Die erfindungsgemäßen Oligomere lassen sich beispielsweise mittels in der Literatur beschriebenen Verfahren durch Umsetzung von Verbindungen der allgemeinen Formel **II** in an sich bekannter Weise (z.B. L. Christensen, R. Fitzpatrick, B. Gildea, K.H. Petersen, H.F. Hansen, T. Koch, M. Egholm, O. Buchaedt, P.E. Nielsen, J. Coull, R.H. Berg, *J.Pept.Sci.* **1995**, 1, 175-183. T. Koch, H.F. Hansen, P. Andersen, T. Larsen, H.G. Batz, K. Otteson, H. Örum, *J.Pept.Res. **1997**,* 49, 80-88. F. Bergmann, W. Bannwarth, S. Tam, *Tetrahedron Lett*. **1995**, 36, 6823-6826) herstellen.

In den Verbindungen der allgemeinen Formel **II** 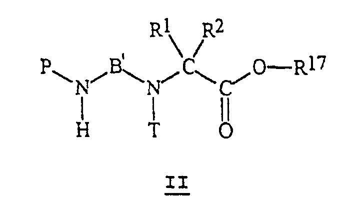 ist B' wie oben definiert,
T ein H-Atom oder eine Gruppe der Formel

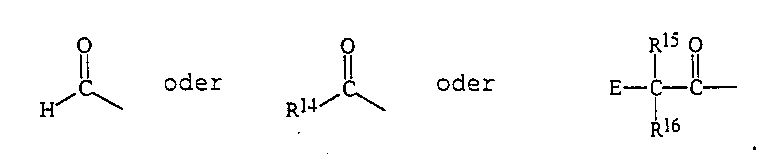

Der Rest R¹⁷ kann ein H-Atom oder ein Allyl-, Benzyl, Ethyl-, Methyl-, 2,2,2-Trichlor-tert.butyl-, 2,2,2-Trichlorethyl-, α-Chloro-(trifluormethyl)benzyl-, 2-(p-Toluolsulfonyl)ethyl-, Diphenylmethyl-, 2-(Trimethylsilyl)ethyl-, Methoxymethyl-, (2-Trimethylsilyl)ethoxymethyl-, Benzyloxymethyl-, oder ein (2-Methoxy)ethyloxymethyl-Rest sein.

Wenn der Rest R¹⁷ kein H-Atom, ist kann er an eine feste Phase gebunden sein. Als feste Phase eignen sich alle konventionellen Festphasenharze, die in der organischen Festphasensynthese angewendet werden, bevorzugt werden Polystyrol-divinylbenzol-, Polyethylenglycol- oder Polyethylen-glycol-polystyrol-Harze.

P kann ein H-Atom oder eine abspaltbare Aminschutzgruppe sein. Die Aminschutzgruppe muß in Gegenwart der Nukleobasen-Schutzgruppen X¹ bis X⁴ selektiv abspaltbar sein. Vorzugsweise ist P ein H-Atom, eine Oxocarbamat- oder eine Thiocarbamat-Schutzgruppe, am stärksten bevorzugt ist P ein H-Atom oder eine Fmoc-, Boc-, Cbz-, Mmt- oder eine Bhoc-Schutzgruppe.

Der Rest R¹⁴ kann eine Gruppe der Formel CHₙX₃₋ₙ (n = 0 bis 3, X = F, Cl, Br, I), Phenyl oder para-Methoxyphenyl sein.

E, die Reste R¹ und R², sowie R¹⁵ und R¹⁶ sind wie oben definiert.

Die Verbindungen der allgemeinen Formel **II** können zum Beispiel nach bekannten Verfahren aus Verbindungen der allgemeinen Formel I hergestellt werden (PCT/EP98/04622).

Die Herstellung von Verbindungen der allgemeinen Formel I geschieht mittels der Ugi-Reaktion (U-4CR) beispielsweise nach folgendem Reaktionsschema:

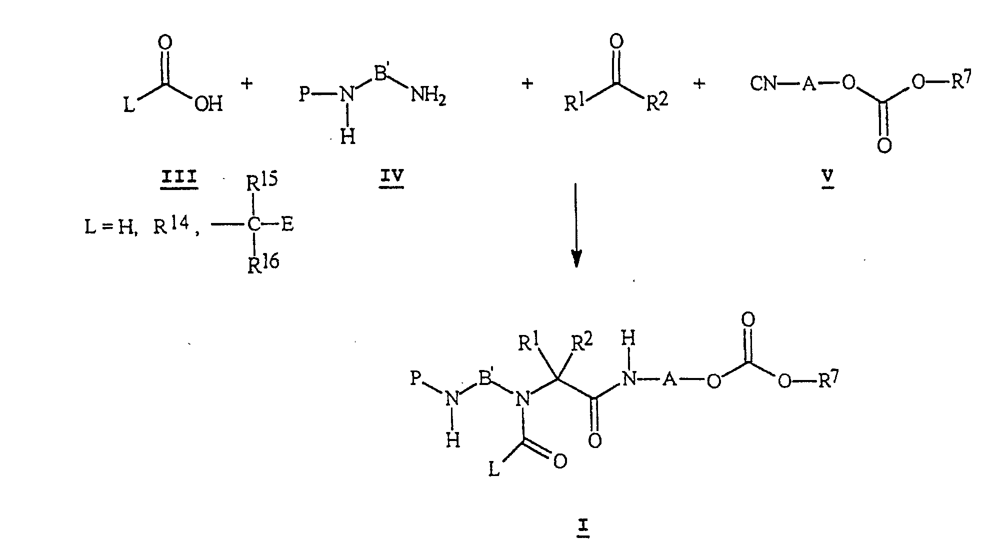

Die Durchführung kann beispielsweise wie in der Literatur beschrieben (I. Ugi et al., Chem. Ber., **1961**, 94, 2802.) erfolgen. Die Nukleinbasen-Essigsäure-Komponenten E-C(R¹⁵R¹⁶)-COOH werden wie in der Literatur beschrieben hergestellt (E. Uhlmann, A. Peyman, G. Breipohl, D.W. Will, *Angew.Chem*, **1998**, 110, 2954-2983) .
Die Aminkomponenten der allgemeinen Formel **IV** werden z. B. entsprechend der Methode von Krapcko hergestellt (A.P. Krapcko, C.S. Kuile, Synthetic Communications, **1990**, 20(16), 2559-2564). Die Isocyanidkomponenten der allgemeinen Formel **V** können nach einem der in Patentanmeldung PCT/EP98/04622 offenbarten Verfahren hergestellt werden. Die Verfahren eignen sich sowohl für harzgebundene Isocyanidkomponenten als auch für nicht harzgebundene Isocyanidkomponenten.

Anschließend werden die Verbindungen der allgemeinen Formel **I** zum Beispiel nach dem in der Literatur beschriebenen Verfahren (Th. Lindhorst, H. Bock, I. Ugi, *Tetrahedron* **1999**, *55*, 7411-7420; PCT/EP98/04622) zu den Verbindungen der allgemeinen Formel **II** umgesetzt. Dies erfolgt zum Beispiel durch Zugabe einer äquimolaren Menge einer nukleophilen Base, wie z.B. Kaliumtert.Butanolat, zu den Verbindungen der allgemeinen Formel **I** in einem aprotischen Lösungsmittel beispielsweise nach folgendem Schema.

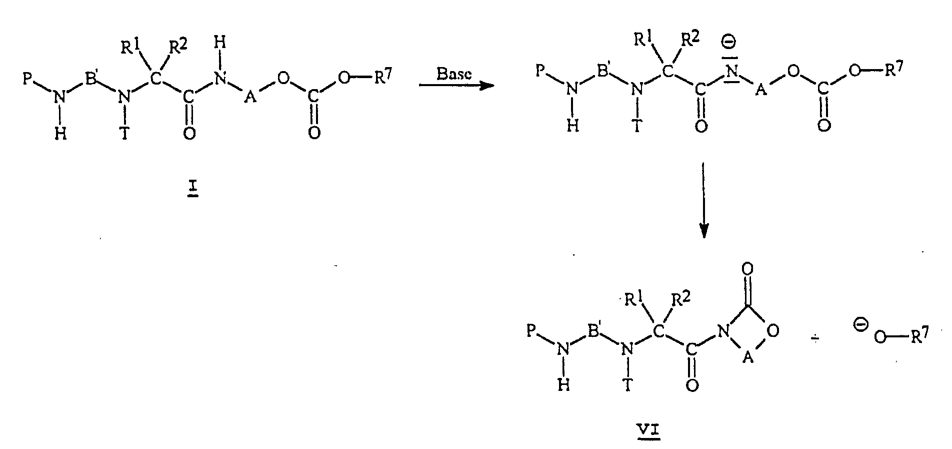

In den Verbindungen der allgemeinen Formel **I** 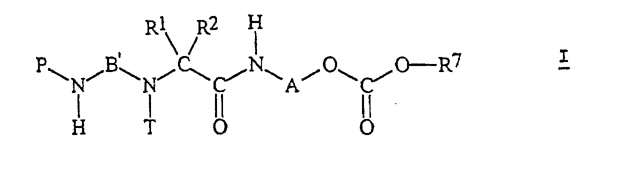 sind die Gruppen B', T, P, sowie die Reste R¹ und R² definiert wie in den Verbindungen der allgemeinen Formel **II**.

Der Rest R⁷ ist definiert wie der Rest R¹⁷ in der Verbindung der allgemeinen Formel **II** oder kann ein Phenyl-Rest sein, darf aber kein H-Atom sein.

A kann eine Gruppe der Formel -C(R³,R⁴)-C(R⁵,R⁶)- sein, wobei die Reste R³ bis R⁶ unabhängig voneinander H-Atome, Phenyl- oder Methylreste sind.

Besonders gut geeignet ist dieses Verfahren zur Generierung von neuartigen PNA-Monomeren, deren Seitenketten denen nicht natürlicher Aminosäuren entsprechen. Bei den bisher bekannten Methoden muß dazu die nichtnatürliche Aminosäure aufwendig hergestellt werden. Nach der basischen Abspaltung der C-terminalen Schutzgruppe kann die basenstabile Schutzgruppe P gegebenenfalls durch eine basenlabile Schutzgruppe P (z. B. Fmoc) ersetzt werden.
Wenn der Rest R⁷ die Nukleophilie des daran gebundenen O-Atoms erniedrigt (R⁷ ist z. B. ein Phenyl-Rest), sind die Intermediate **VI** isolierbar (siehe Patentanmeldung PCT/EP98/04622). **VI** kann anschließend durch milde basische Hydrolyse in die Verbindungen der allgemeinen Formel **II**, wobei R¹⁷ ein H-Atom ist, überführt werden.
Wenn in den Verbindungen der allgemeinen Formel **I** der Rest R⁷ die Nukleophilie des daran gebundenen O-Atoms nicht erniedrigt, sind die Intermediate **VI** nicht isolierbar. In diesen Fällen setzt sich **VI** in situ mit dem durch den intramolekularen Ringschluß gebildeten Alkoholation zum entsprechenden Ester der allgemeinen Formel **II** beispielhaft nach folgendem Schema um.

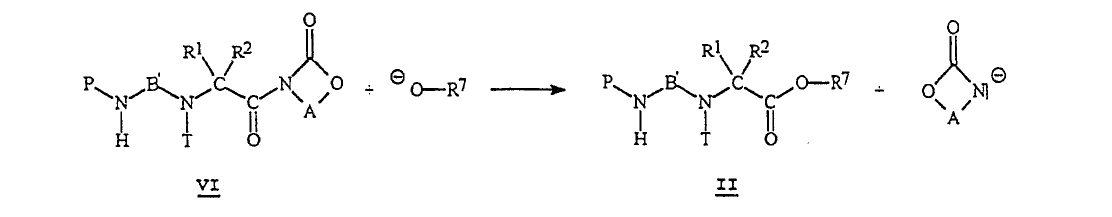

Nach der basischen Abspaltung der C-terminalen-Schutzgruppe ist es möglich, eine basenstabile, wie vorstehend definierte Schutzgruppe P (z.B. Boc) in den Verbindungen der allg. Formel **II** durch gängige Methoden zu entfernen und gegebenenfalls durch eine neue, in Gegenwart der Nukleobasen-Schutzgruppen X¹ bis X⁴ selektiv abspaltbare Schutzgruppe (z.B. die basenlabile Fmoc-Schutzgruppe) zu ersetzen.

### Beispiele:

### Beispiel 1: Herstellung von

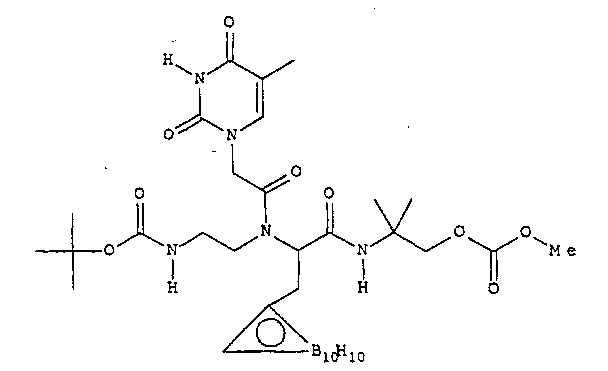

Jeweils 5 mmol Thyminylessigsäure, 2-(1,2-Dicarba-closododecaboran)-ethanal, N-Boc-ethylendiamin und 2-Isocyano-2,2-(dimethyl)ethyl-kohlensäuremethylester werden in 50 ml Trifluorethanol gelöst und bei 25 °C gerührt. Nach Beendigung der Reaktion wird das Lösungsmittel entfernt.
Das Reaktionsgemisch wird durch Säulenchromatographie gereinigt. Man erhält das Reaktionsprodukt in 70%-iger Ausbeute.

### Beispiel 2: Herstellung von

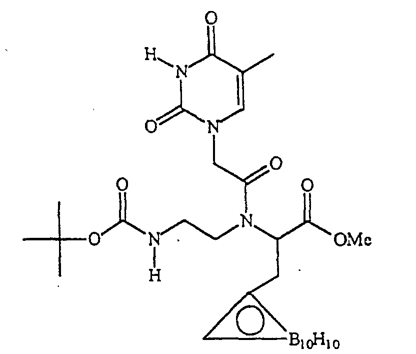

2 mmol Reaktionsprodukt aus Beispiel 1 werden in 10 ml absolutem THF gelöst und bei 25 °C 2 mmol Natriumhydrid zugegeben. Nach Beendigung der Reaktion wird das Reaktionsgemisch über eine kurze Kieselgelsäule filtriert. Das Lösungsmittel wird entfernt und das Reaktionsprodukt durch Säulenchromatographie gereinigt. Man erhält das Reaktionsprodukt in 70%iger Ausbeute.

### Beispiel 3: Herstellung von

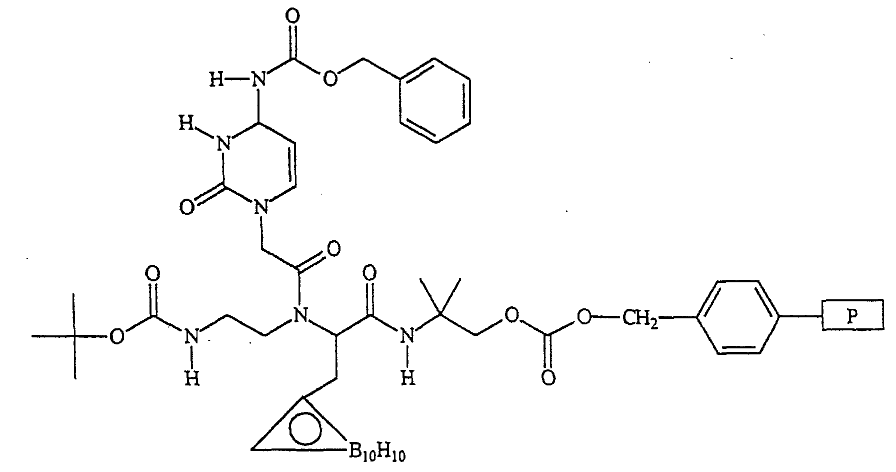

Jeweils 5 mmol , (N⁴-Cbz-Cytosyl)essigsäure, 2-(1,2-Dicarbacloso-dodecaboran)-ethanal, N-Boc-ethylendiamin und 2-Isocyano-2,2-(dimethyl)ethyl-kohlensäuremethylpolystyrolharz-ester werden in 50 ml Trifluorethanol suspendiert und bei 25 °C gerührt. Nach Beendigung der Reaktion wird das Lösungsmittel über eine Fritte entfernt und das Reaktionsgemisch mehrmals mit Methanol, Methylenchlorid, einer auf pH = 9 eingestellten Natriumhydrogencarbonat-Lösung und Wasser gewaschen.
Man erhält das Reaktionsprodukt in 80%-iger Ausbeute (ermittelt durch bromometrische Bestimmung von nicht umgesetztem Isocyanid-Harz).

### Beispiel 4: Herstellung von

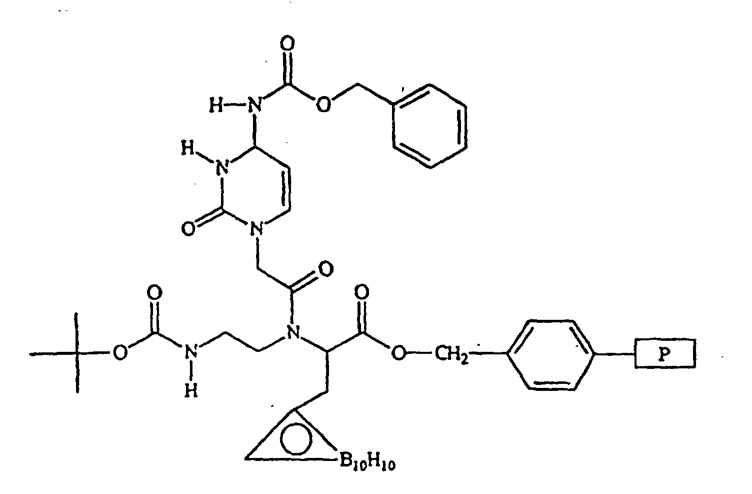

2 mmol Reaktionsprodukt aus Beispiel 3 werden in 10 ml absolutem THF suspendiert und bei 25 °C 2 mmol Kalium-tert.Butanolat zugegeben. Nach Beendigung der Reaktion wird das Lösungsmittel über eine Fritte entfernt und das Reaktionsgemisch mehrmals mit Methanol, Methylenchlorid, einer auf pH = 9 eingestellten Natriumhydrogencarbonat-Lösung und Wasser gewaschen.
Man erhält das Reaktionsprodukt in 60%iger Ausbeute.

### Beispiel 5: Herstellung von

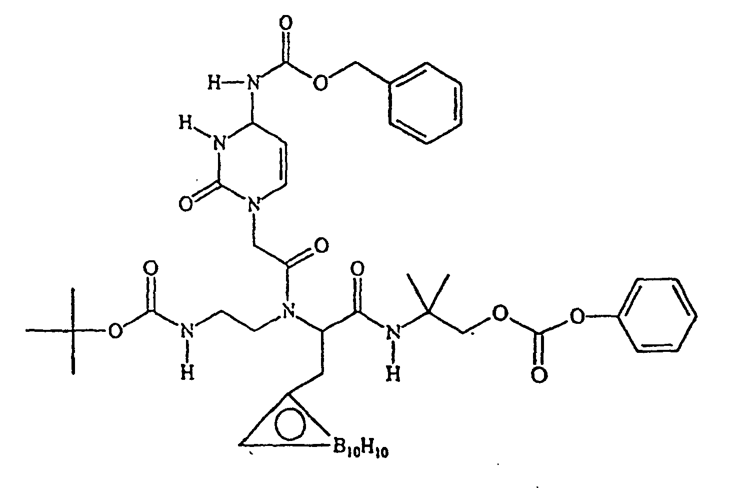

Jeweils 5 mmol (N⁴-Cbz-Cytosyl)essigsäure, 2-(1,2-Dicarba-closododecaboran)-ethanal, N-Boc-ethylendiamin und 2-Isocyano-2,2-(dimethyl)ethyl-kohlensäurephenylylester werden in 50 ml Trifluorethanol gelöst und bei 25 °C gerührt. Nach Beendigung der Reaktion wird das Lösungsmittel entfernt.
Das Reaktionsgemisch wird durch Säulenchromatographie gereinigt. Man erhält das Reaktionsprodukt in 80%-iger Ausbeute.

### Beispiel 6: Herstellung von

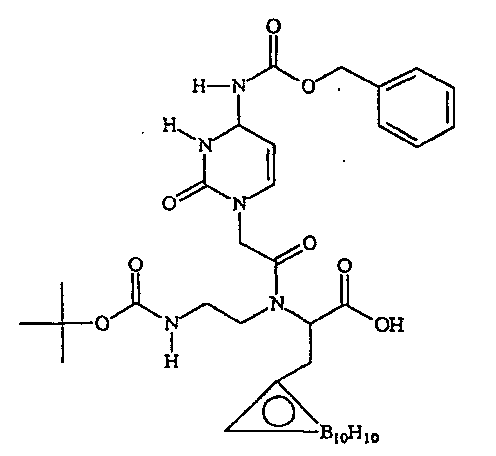

2 mmol Reaktionsprodukt aus Beispiel 5 werden in 10 ml absolutem THF gelöst und bei 25 °C 2 mmol Kalium-tert.Butanolat zugegeben. Nach Beendigung der Reaktion wird das Reaktionsgemisch mit einer wäßrigen 1 molaren Kaliumhydroxid-Lösung versetzt und gerührt bis sich kein Reaktionsumsatz mehr feststellen läßt. Die Reaktionslösung wird neutralisiert und das Lösungsmittel entfernt. Das Reaktionsprodukt wird durch Säulenchromatographie gereinigt. Man erhält das Reaktionsprodukt in 70%iger Ausbeute.

### Beispiel 7: Herstellung von

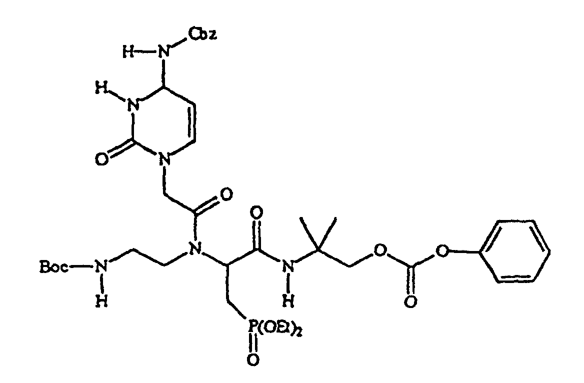

Jeweils 5 mmol (N⁴-Cbz-Cytosyl)essigsäure, 2-Phosphonsäurediethylester-ethanal, N-Boc-ethylendiamin und 2-Isocyano-2,2-(dimethyl)ethyl-kohlensäurephenylester werden in 50 ml Ethanol gelöst. Zur Verbesserung der Löslichkeitseigenschaften von (N⁴-Cbz-Cytosyl)essigsäure werden 5 mmol Triethylamin hinzugefügt und anschließend bei 25 °C gerührt. Nach Beendigung der Reaktion wird das Lösungsmittel entfernt.
Das Reaktionsgemisch wird durch Säulenchromatographie gereinigt. Man erhält das Reaktionsprodukt in 70%-iger Ausbeute.

### Beispiel 8: Herstellung von

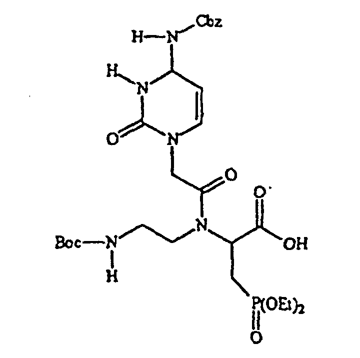

2 mmol Reaktionsprodukt aus Beispiel 7 werden in 10 ml absolutem THF gelöst und bei 25 °C 2 mmol Kalium-tert.Butanolat zugegeben. Nach Beendigung der Reaktion wird das Reaktionsgemisch mit 2 mmol Kaliumhydroxid einer wäßrigen 1 molaren Lösung versetzt und gerührt bis sich kein Reaktionsumsatz mehr feststellen läßt. Die Reaktionslösung wird neutralisiert und das Lösungsmittel entfernt. Das Reaktionsprodukt wird durch Säulenchromatographie gereinigt. Man erhält das Reaktionsprodukt in 55%iger Ausbeute.

### Beispiel 9: Herstellung von

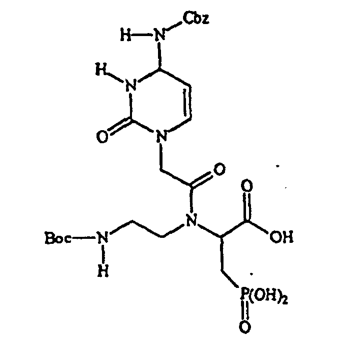

2 mmol Reaktionsprodukt aus Beispiel 8 werden in 10 ml absolutem THF gelöst und bei 50 °C mit 2 mmol Kaliumhydroxid einer wäßrigen 1 molaren Lösung versetzt. Nach Beendigung der Reaktion wird die Reaktionslösung neutralisiert und das Lösungsmittel entfernt. Das Reaktionsprodukt wird durch präparative HPLC gereinigt. Man erhält das Reaktionsprodukt in 40%iger Ausbeute.

### Beispiel 10: Herstellung von

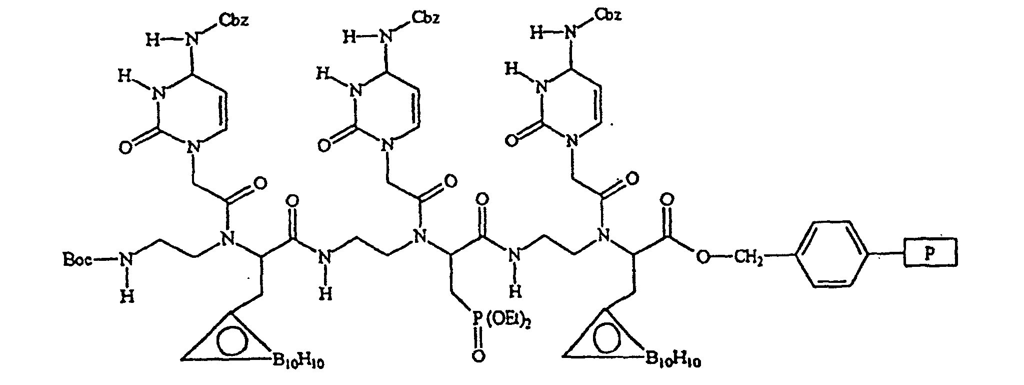

### Syntheseprotokoll:

Schritt 1: 100 mg Reaktionsprodukt aus Beispiel 4 werden in Methylenchlorid 12 h vorgequellt.
Schritt 2: tert.Butyloxycarbonyl-Entschützung am Peptidsyntheziser mit einer 50%-igen Lösung aus Trifluoressigsäure in Methylenchlorid (1:1 v/v, 2 ml, 1 x 2 Minuten, 1 x 30 min).
Schritt 3: Waschen mit Methylenchlorid (2 ml, 4 x 20 Sekunden).
Schritt 4: Neutralisation mit DIPEA/Methylenchlorid (1:19 v/v, 2 ml, 2 x 3 min).
Schritt 5: Waschen mit Methylenchlorid (2 ml, 2 x 20 Sekunden), waschen mit DMF (2 ml, 3 x 20 Sekunden).
Schritt 6: Zufügen von 4 Äquivalenten HBTU und Diethylcyclohexylamin in DMF/Pyridin (1:1 v/v) und 4 Äquivalenten Reaktionsprodukt aus Beispiel 8.
Schritt 7: Waschen mit DMF (2 ml, 3 x 20 Sekunden) und Methylenchlorid (3 ml, 3 x 20 Sekunden).
Schritt 8: Capping mit einer Lösung aus 0,5 M Essigsäureanhydrid/0,5 M DMF
Schritt 9: Waschen mit DMF (2 ml, 3 x 20 Sekunden) und Methylenchlorid (3 ml, 3 x 20 Sekunden).
Schritt 10: Wiederholen des Synthesezyklus ab Schritt 2, in Schritt 6 werden 4 Äquivalente Reaktionsprodukt aus Beispiel 6 statt Reaktionsprodukt aus Beispiel 8 eingesetzt.
Schritt 11: Trocknen im Stickstoffstrom.

Man erhält das Reaktionsprodukt in 97%-iger Ausbeute.

### Beispiel 11: Herstellung von

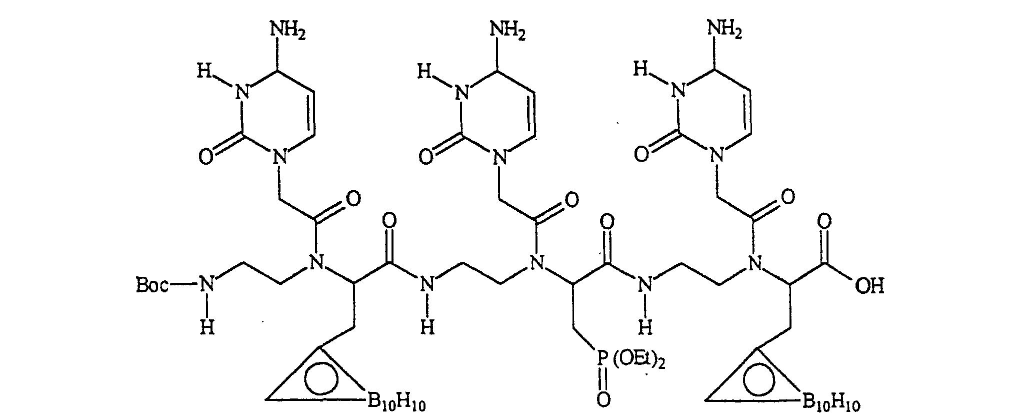

Das Reaktionsprodukt aus Beispiel 10 wird in Methanol suspendiert und eine katalytische Menge Platin auf Kohlenstoff zugefügt. Das Reaktionsgemisch wird in einer Wasserstoffatmosphäre hydriert.
Nach Beendigung der Reaktion wird das Lösungsmittel entfernt und das Produkt durch präparative HPLC gereinigt. Man erhält das Reaktionsprodukt in 96%iger Ausbeute.

### Beispiel 12: Herstellung von

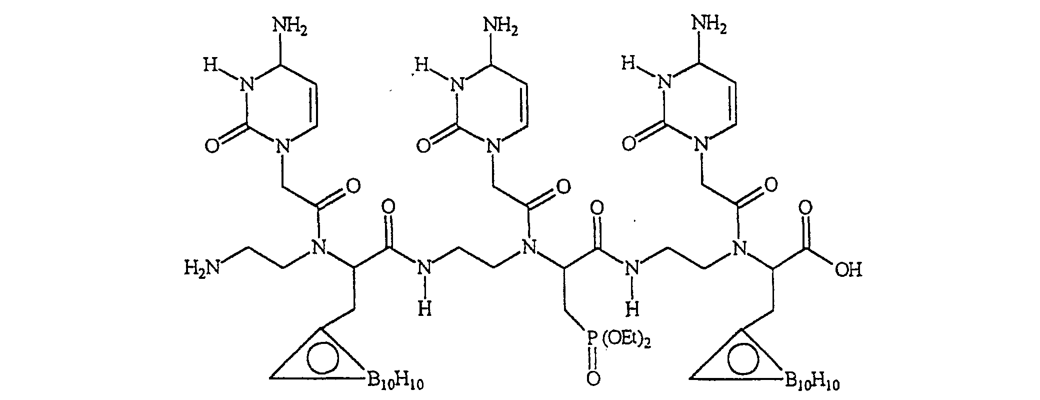

Das Reaktionsprodukt aus Beispiel 11 wird in Methylenchlorid suspendiert. Es werden jeweils 1 ml Trifluoressigsäure und Thiophenol zugefügt. Nach Beendigung der Reaktion wird das Reaktionsprodukt durch präparative HPLC gereinigt. Man erhält das Reaktionsprodukt in 99%iger Ausbeute.

## Patentansprüche

1. Verbindungen der Formel
W-U-Z
wobei W ein H-Atom, eine Aminosäure- oder PNA-Einheit ist,
U mindestens eine Einheit der Formel Y und gegebenenfalls eine oder mehrere Aminosäure- und/oder PNA-Einheiten enthält,
Z eine OH-Funktion, eine Aminosäure-, oder PNA-Einheit ist,
Y eine Einheit der Formel 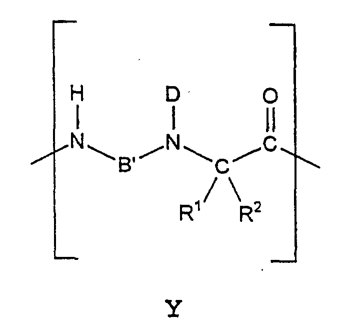 ist, worin
B' eine Gruppe der Formel, 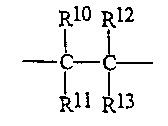 D eine Gruppe der Formel, 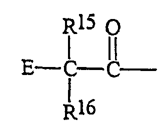 ist,
die Reste R¹⁰ bis R¹³ jeweils unabhängig voneinander bis zu 20 C-Atome umfassen und unabhängig voneinander H-Atome, unsubstituierte Alkyl-, Alkenyl-, Alkaryl-, Aryl-, oder alicyclische Reste sind, wobei die Reste verzweigt oder unverzweigt sind, und optional jeweils zwei der Reste R¹⁰ bis R¹³, die durch bis zu zwei Kohlenstoffatome voneinander getrennt sind, Bestandteile eines gemeinsamen Ringsystems sind, wobei dieses Ringsystem ein unsubstituierter oder mit einem verzweigten oder unverzweigten C₁-C₅ Alkylrest substituierter alicyclischer Monocyclus (3-8 Ringatome) oder ein Phenyl-Ring ist,
die Reste R¹⁵ und R¹⁶ jeweils unabhängig voneinander bis zu 20 C-Atome umfassen und unabhängig voneinander H-Atome, unsubstituierte Alkyl-, Alkenyl-, Alkaryl-, Aryl-, oder alicyclische Reste sind, wobei die Reste verzweigt oder unverzweigt sind, und optional die Reste R¹⁵ und R¹⁶ Bestandteile eines gemeinsamen Ringsystems sind, wobei dieses Ringsystem ein unsubstituierter oder mit einem verzweigten oder unverzweigten C₁-C₅ Alkylrest substituierter alicyclischer Monocyclus (3-6 Ringatome) ist,
E eine natürliche oder nichtnatürliche gegebenenfalls mit Schutzgruppen substituierte, zur Watson-Crick- oder Hoogsteen-Basenpaarung fähige Nukleobase ist,
die Reste R¹ und R² jeweils unabhängig voneinander H-Atome, Alkyl-, Alkenyl-, Alkaryl-, Aryl-, oder alicyclische Reste mit bis zu 20 C-Atomen sind, wobei mindestens einer der Reste R¹ und R² eine oder mehrere Phosphonsäureester-, Phosphonsäure- oder Carbaboran-Funktionen aufweist.

2. Verbindungen nach Anspruch 1, **dadurch gekennzeichnet, daß** sie insgesamt aus bis zu 50 dieser Einheiten W, U und Z bestehen.

3. Verbindungen nach Anspruch 1 oder 2, **dadurch gekennzeichnet, daß** W ein H-Atom, U eine oder mehrere Einheiten der Formel Y und Z eine OH-Gruppe ist.

4. Verbindungen nach einem der vorstehenden Ansprüche, worin mindestens einer der Reste R¹ oder R² eine oder mehrere Phosphonsäureester- oder Phosphonsäure-Funktionen aufweist.

5. Verbindungen nach einem der vorstehenden Ansprüche, worin mindestens einer der Reste R¹ oder R² eine oder mehrere Carbaboran-Funktionen aufweist.

6. Verbindungen der allgemeinen Formel **II** 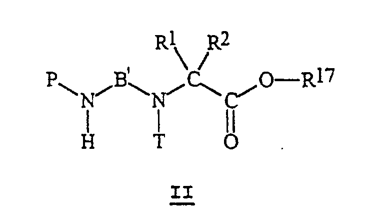 ,worin T ein H-Atom oder eine Gruppe der Formel 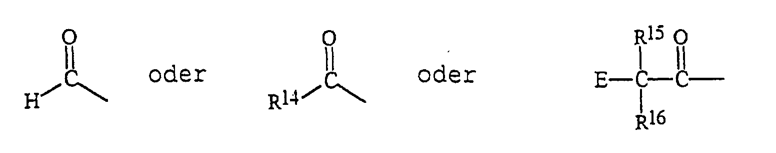 ist,
der Rest R¹⁷ ein H-Atom oder ein Allyl-, Benzyl, Ethyl-, Methyl-, 2,2,2-Trichlor-tert.butyl-, 2,2,2-Trichlorethyl-, α-Chloro-(trifluormethyl)benzyl-, 2-(p-Toluolsulfonyl)ethyl-, Diphenylmethyl-, 2-(Trimethylsilyl)ethyl-, Methoxymethyl-, (2-Trimethylsilyl)ethoxymethyl-, Benzyloxymethyl-, oder ein (2-Methoxy)ethyloxymethyl-Rest ist,
der Rest P ein H-Atom oder Aminschutzgruppe ist,
der Rest R¹⁴ eine Gruppe der Formel CHₙX₃₋ₙ (n = 0 bis 3, X = F, Cl, Br, I), eine Phenyl- oder para-Methoxyphenyl-Gruppe ist,
B', E, die Reste R¹ und R², sowie R¹⁵ und R¹⁶ wie in Anspruch 1 bis 5 definiert sind.

7. Verbindungen nach Anspruch 6, wobei der Rest R¹⁷ kein H-Atom ist und an eine feste Phase gebunden ist.

8. Verbindungen nach Anspruch 6 oder 7, wobei die Aminschutzgruppe eine Fmoc-, Boc-, Cbz-, Mmt- oder eine Bhoc-Schutzgruppe ist.

9. Verfahren zur Herstellung von Verbindungen nach einem der Ansprüche 1 bis 5, **dadurch gekennzeichnet daß** Verbindungen nach den Ansprüchen 6 bis 8 in an sich bekannter Weise umgesetzt werden.

10. Verwendung von Verbindungen nach einem der Ansprüche 1 bis 5 zur Herstellung eines medikaments zur Krebstherapie.

## Claims

1. **A compound of the formula**
W-U-Z
in which W is a hydrogen atom, an amino acid unit, or a PNA unit,
U contains at least one unit of the formula Y and, optionally, one or more amino acid and/or PNA units,
Z is an OH function, an amino acid unit, or a PNA unit,
Y is a unit of the formula 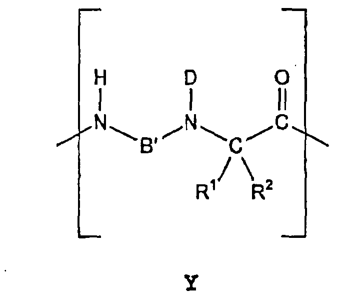 in which
B' is a group of the formula, 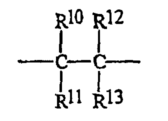 D is a group of the formula 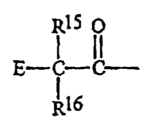 the residues R¹⁰ to R¹³ independently contain up to 20 carbon atoms and independently denote hydrogen atoms or unsubstituted alkyl, alkenyl, alkaryl, aryl, or alicyclic groups, said groups being branched or unbranched, and optionally two each of the residues R¹⁰ to R¹³, separated from each other by up to two carbon atoms, are components of a common ring system, which ring system is either an alicyclic monocyclic compound (3-8 ring atoms), optionally substituted by a branched or unbranched C₁₋₅ alkyl group, or a phenyl ring,
the residues R¹⁵ and R¹⁶ independently contain up to 20 carbon atoms and independently denote hydrogen atoms or unsubstituted alkyl, alkenyl, alkaryl, aryl, or alicyclic groups, said groups being branched or unbranched, and optionally the residues R¹⁵ and R¹⁶ are components of a common ring system, which ring system is an alicyclic monocyclic compound (3-6 ring atoms), optionally substituted by a branched or unbranched C₁₋₅ alkyl group,
E is a natural or synthetic nucleobase, optionally substituted by protecting groups and capable of forming Watson-Crick or Hoogsteen base pairs, and
the residues R¹ and R² are independently hydrogen atoms, alkyl, alkenyl, alkaryl, aryl, or alicyclic groups containing up to 20 carbons, whilst at least one of the residues R¹ and R² exhibits one or more phosphite ester, phosphonic acid, or carbaborane functions.

2. A compound as defined in claim 1, **characterised in that** it consists of a total of up to 50 of the said units W, U and Z.

3. A compound as defined in claim 1 or claim 2, wherein W is a hydrogen atom, U one or more units of formula Y, and Z an OH group.

4. A compound as defined in any of the previous claims, wherein at least one of the residues R¹ and R² exhibits one or more phosphite ester or phosphonic acid functions.

5. A compound as defined in any of the previous claims, wherein at least one of the residues R¹ and R² exhibits one or more carbaborane functions.

6. A compound of the general formula **II** 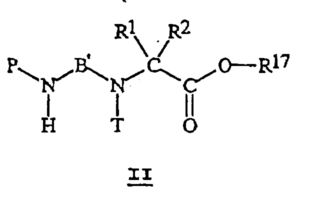 in which T is hydrogen or a group of the formula 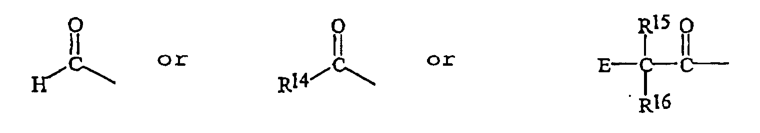 the residue R¹⁷ is hydrogen or allyl, benzyl, ethyl, methyl, 2,2,2-trichloro-tert-butyl, 2,2,2-trichloroethyl, α-chloro-(trifluoromethyl)benzyl, 2-(p-toluenesulfonyl)ethyl, diphenylmethyl, 2-(trimethylsilyl)ethyl, methoxymethyl, (2-trimethylsilyl)ethoxymethyl, benzyloxymethyl, or (2-methoxy)ethyloxymethyl,
the residue P is hydrogen or an amine protecting group,
the residue R¹⁴ is a group of the formula CHₙX₃₋ₙ (n = 0 to 3, X = F, Cl, Br, I), a phenyl group, or a *p*-methoxyphenyl group, and
B' , E, the residues R¹ and R² as well as R¹⁵ and R¹⁶ have the meanings stated in claims 1 to 5.

7. A compound as defined in claim 6, wherein the residue R¹⁷ is not a hydrogen atom and is bound to a solid phase.

8. A compound as defined in claim 6 or claim 7, wherein the amine protecting group is an Fmoc, Boc, Cbz, Mmt, or Bhoc protecting group.

9. A process for the production of a compound as defined in any of claims 1 to 5, wherein compounds as defined in any of claims. 6 to 8 are converted in known manner.

10. Use of compounds according to any of claims 1 to 5 for the preparation of a medicament for cancer therapy.

## Revendications

1. Composés de formule
W-U-Z
dans laquelle W représente un atome d'hydrogène, un acide aminé ou un fragment d'APN,
U contient au moins un motif de formule Y et, le cas échéant, un ou plusieurs acides aminés et/ou fragments d'APN,
Z représente un groupe -OH, un acide aminé ou un fragment d'APN,
Y représente un motif de formule 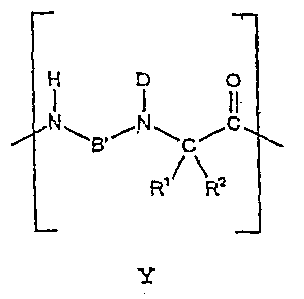 dans laquelle
B' représente un groupe de formule 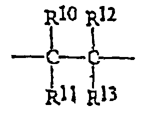 D représente un groupe de formule 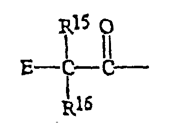 R¹⁰ à R¹³ comportent chacun, indépendamment les uns des autres, jusqu'à 20 atomes de carbone et représentent, indépendamment les uns des autres, un atome d'hydrogène, un groupe alkyle, alkenyle, alkaryle, aryle ou alicyclique sans substituant, les groupes étant ramifiés ou non ramifiés, et deux des groupes R¹⁰ à R¹³, séparés par au plus deux atomes de carbone, pouvant éventuellement être des constituants d'un système cyclique commun, ledit système cyclique étant un monocycle (3-8 membres) alicyclique sans substituant ou portant un substituant alkyle en C₁-C₅ ramifié ou non ramifié ou un phényle,
R¹⁵ et R¹⁶ comportent chacun, indépendamment l'un de l'autre, jusqu'à 20 atomes de carbone et représentent, indépendamment l'un de l'autre, un atome d'hydrogène, un groupe alkyle, alkenyle, alkaryle, aryle ou alicyclique sans substituant, les groupes étant ramifiés ou non ramifiés, et
R¹⁵ et R¹⁶ pouvant éventuellement être des constituants d'un système cyclique commun, ledit système cyclique étant monocyclique (3-6 membres) alicyclique sans substituant ou portant un substituant alkyle en C₁-C₅ ramifié ou non ramifié,
E représente une base nucléique, naturelle ou non, capable d'appariement de type Watson-Crick ou Hoogsteen, portant le cas échéant un substituant de protection,
R¹ et R² représentent chacun, indépendamment l'un de l'autre, un atome d'hydrogène, un groupe alkyle, alkenyle, alkaryle, aryle ou alicyclique comportant jusqu'à 20 atomes de carbone, et au moins un des groupes R¹ ou
R² comportant une ou plusieurs fonctions ester d'acide phosphonique, acide phosphonique ou carbaborane.

2. Composés selon la revendication 1, **caractérisés en ce qu'**ils sont composés d'au plus 50 fragments W, U et Z.

3. Composés selon la revendication 1 ou 2 **caractérisés en ce que** W représente un atome d'hydrogène, U représente un ou plusieurs motifs de formule Y et Z représente un groupe -OH.

4. Composés selon l'une quelconque des revendications précédentes, **caractérisés en ce que** au moins un des groupes R¹ ou R² comporte une ou plusieurs fonctions ester d'acide phosphonique ou acide phosphonique.

5. Composés selon l'une quelconque des revendications précédentes, **caractérisés en ce que** au moins un des groupes R' ou R² comporte une ou plusieurs fonctions carbaborane.

6. Composés de formule générale II 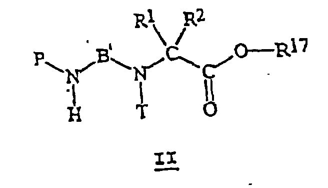 dans laquelle T représente un atome d'hydrogène ou un groupe de formule 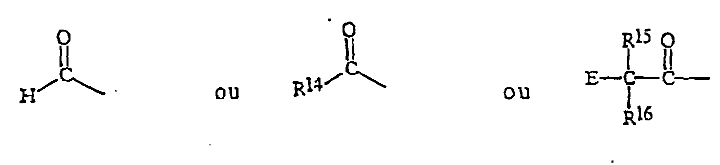 R¹⁷ représente un atome d'hydrogène ou un groupe allyle, benzyle, éthyle, méthyle, 2,2,2-trichloro-tert-butyle, 2,2,2-trichloroéthyle, α-chloro-(trifluorométhyl)benzyle, 2-(p-toluènesulfonyl)éthyle, diphényl-méthyle, 2-(triméthylsilyl)éthyle, méthoxyméthyle, (2-triméthyl-silyl)éthoxyméthyle, benzyloxyméthyle ou (2-méthoxy)éthyloxyméthyle,
P représente un atome d'hydrogène ou un groupe de protection d'amine,
R¹⁴ représente un groupe de formule CHₙX₃₋ₙ (n = 0 à 3, X = F, Cl, Br, I), phényle ou para-méthoxyphényle,
B', E, R¹ et R², ainsi que R¹⁵ et R¹⁶ sont définis tels que dans les revendications 1 à 5.

7. Composés selon la revendication 6, **caractérisés en ce que** R¹⁷ ne représente pas un atome d'hydrogène et est lié à une phase solide.

8. Composés selon la revendication 6 ou 7, **caractérisés en ce que** le groupe de protection d'amine est un groupe de protection Fmoc, Boc, Cbz, Mmt ou Bhoc.

9. Procédé pour la préparation de composés selon l'une quelconque des revendications 1 à 5, **caractérisé en ce que** l'on fait réagir les composés selon les revendications 6 à 8 par des méthodes connues.

10. Utilisation des composés selon l'une quelconque des revendications 1 à 5, pour la préparation de médicaments utilisés dans les traitements du cancer.
